# EUROPÄISCHE PATENTSCHRIFT

(11) **EP 3 389 467 B1**
(45) Veröffentlichungstag und Bekanntmachung des Hinweises auf die Patenterteilung: **25.03.2020**
(21) Anmeldenummer: 16825362.3
(22) Anmeldetag: 16.12.2016
(51) Int. Cl.: A61B 1/00, A61B 17/34, A61B 1/12

(54) **ENDOSKOPIECHIRURGIEINSTRUMENT**
ENDOSCOPIC SURGICAL INSTRUMENT
INSTRUMENT DE CHIRURGIE ENDOSCOPIQUE

(30) Priorität: 18.12.2015 DE 202015106940 U
(43) Veröffentlichungstag der Anmeldung: 24.10.2018
(73) Patentinhaber: DANNORITZER Medizintechnik GmbH & Co. KG, 78532 Tuttlingen (DE)
(72) Erfinder: ARLT, Matthias, 78576 Neuhausen ob Eck (DE)
(74) Vertreter: Daub, Thomas
(86) Internationale Anmeldenummer: PCT/EP2016/081592
(87) Internationale Veröffentlichungsnummer: WO 2017/103217

(56) Entgegenhaltungen:
- EP-A1- 2 095 757
- WO-A1-2015/179837
- JP-A- 2009 201 563
- US-A- 5 575 756

## Beschreibung

### Stand der Technik

Die Erfindung betrifft ein Endoskopiechirurgieinstrument mit einem Endoskopieschaft und mit einer Saug- und/oder Spüleinheit.

Es ist bereits vorgeschlagen worden, dass das Endoskopiechirurgieinstrument eine Sicherungsmutter umfasst, die zu einer Sicherung des Endoskopieschafts an der Saug- und/oder Spüleinheit vorgesehen ist.

Ferner ist aus JP 2009 201563 A und EP 2 095 757 A1 jeweils bereits ein Endoskopiechirurgieinstrument mit einem Endoskopieschaft, mit einer Saug- und/oder Spüleinheit, mit einem Sicherungselement, das dazu vorgesehen ist, den zumindest einen Endoskopieschaft an der zumindest einen Saug- und/oder Spüleinheit in einem gekoppelten Zustand zu sichern, und mit einer Befestigungseinheit, die zu einer verliersicheren Befestigung des zumindest einen Sicherungselements an der zumindest einen Saug- und/oder Spüleinheit vorgesehen ist, bekannt.

Zudem ist aus US 5 575 756 A bereits ein Endoskopiechirurgieinstrument mit einem abnehmbaren Okularadapter bekannt.

Die Aufgabe der Erfindung besteht insbesondere darin, ein gattungsgemäßes Endoskopiechirurgieinstrument mit verbesserten Eigenschaften hinsichtlich einer Befestigung des Endoskopieschafts an der Saug- und/oder Spüleinheit bereitzustellen, wobei insbesondere ein Verlust der Sicherungsmutter in einem demontierten Zustand des Endoskopiechirurgieinstruments verhindert werden soll. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einem Endoskopiechirurgieinstrument mit zumindest einem Endoskopieschaft, mit zumindest einer Saug- und/oder Spüleinheit, mit zumindest einem Sicherungselement, das dazu vorgesehen ist, den zumindest einen Endoskopieschaft an der zumindest einen Saug- und/oder Spüleinheit in einem gekoppelten Zustand zu sichern, und mit zumindest einer Befestigungseinheit, die zu einer verliersicheren Befestigung des zumindest einen Sicherungselements an der zumindest einen Saug- und/oder Spüleinheit vorgesehen ist.

Es wird vorgeschlagen, dass die zumindest eine Befestigungseinheit zumindest ein Abdeckelement umfasst, das das zumindest eine Sicherungselement in einem montierten Zustand zumindest teilweise umschließt, wobei das zumindest eine Abdeckelement zumindest eine Bedienausnehmung zur Bedienung des zumindest einen Sicherungselements aufweist. Dadurch kann auf konstruktiv einfache und vorteilhaft kostengünstige Weise eine bevorzugt zuverlässige Verliersicherung des zumindest einen Sicherungselements in einem demontierten Zustand des Endoskopiechirurgieinstruments, insbesondere zu einer Reinigung, erreicht werden.

Unter einer "Saug- und/oder Spüleinheit" soll in diesem Zusammenhang insbesondere eine Einheit zumindest zu einem Spülen, insbesondere mit einer Flüssigkeit, und/oder zu einem Absaugen von Flüssigkeit bei einem chirurgischen Eingriff verstanden werden. Die Saug- und/oder Spüleinheit ist vorzugsweise mit zumindest einem Schlauch, insbesondere einem Saugschlauch und/oder einem Spülschlauch, koppelbar ausgebildet. Die Saug- und/oder Spüleinheit ist bevorzugt über den zumindest einen Schlauch mit zumindest einer externen Pumpe koppelbar ausgebildet. Unter "vorgesehen" soll insbesondere speziell ausgestaltet, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Unter "sichern" soll in diesem Zusammenhang insbesondere verstanden werden, dass eine Bewegung der zumindest einen Saug- und/oder Spüleinheit relativ zu dem zumindest einen Endoskopieschaft in zumindest einer Richtung verhindert wird. Vorzugsweise ist das zumindest eine Sicherungselement dazu vorgesehen, die zumindest eine Saug- und/oder Spüleinheit in Längsrichtung des zumindest einen Endoskopieschafts, insbesondere gegen ein Verrutschen, zu sichern. In einem montierten Zustand verhindert das zumindest eine Sicherungselement eine Relativbewegung zwischen der zumindest einen Saug- und/oder Spüleinheit und dem zumindest einen Endoskopieschaft parallel zur Längsrichtung des zumindest einen Endoskopieschafts zumindest nahezu vollständig. Das zumindest eine Sicherungselement ist vorzugsweise zu einer formschlüssigen Sicherung vorgesehen. Es ist jedoch auch denkbar, dass das zumindest eine Sicherungselement alternativ oder zusätzlich zu einer kraftschlüssigen Sicherung vorgesehen ist. Das zumindest eine Sicherungselement ist besonders bevorzugt zu einer, insbesondere manuell und werkzeuglos, lösbaren Sicherung der Saug- und/oder Spüleinheit relativ zu dem zumindest einen Endoskopieschaft vorgesehen.

Ferner wird vorgeschlagen, dass das zumindest eine Sicherungselement als Sicherungsmutter ausgebildet ist. Dadurch kann eine bevorzugt kostengünstige, bevorzugt kompakte und konstruktiv einfache Ausgestaltung des zumindest einen Sicherungselements erreicht werden. Es sind jedoch auch andere, einem Fachmann als sinnvoll erscheinende Ausgestaltungen des zumindest einen Sicherungselements, wie beispielsweise als Sicherungsschraube, als Klemmelement, als Bajonettelement und/oder als Sicherungsstift, denkbar. Die Sicherungsmutter umfasst ein Innengewinde und eine Betätigungsaußenfläche, die vorzugsweise werkzeuglos und manuell durch einen Bediener des Endoskopiechirurgieinstruments betätigbar ausgebildet ist. In einem bevorzugten Ausführungsbeispiel ist die Betätigungsaußenfläche der Sicherungsmutter zumindest teilweise geriffelt ausgebildet. Es sind jedoch auch andere, einem Fachmann als sinnvoll erscheinende Ausgestaltungen der Betätigungsaußenfläche der Sicherungsmutter, wie beispielsweise als Außensechskant, denkbar.

Dadurch, dass die zumindest eine Befestigungseinheit zumindest ein Abdeckelement umfasst, das das zumindest eine Sicherungselement in einem montierten Zustand zumindest teilweise umschließt, kann eine vorteilhaft einfache und bevorzugt zuverlässige Verliersicherung des zumindest einen Sicherungselements erreicht werden. Das zumindest eine Abdeckelement umschließt das zumindest eine Sicherungselement in einem montierten Zustand zumindest teilweise in zumindest einer Ebene, die vorzugsweise senkrecht zu einer Richtung, in der das zumindest eine Sicherungselement die zumindest eine Saug- und/oder Spüleinheit relativ zu dem zumindest einen Endoskopieschaft in einem montierten Zustand sichert, angeordnet ist. Vorzugsweise umfasst das zumindest eine Abdeckelement zumindest zwei Bereiche, die insbesondere beabstandet zueinander und in einem montierten Zustand der zumindest einen Befestigungseinheit vorzugsweise gleichmäßig verteilt um das zumindest eine Sicherungselement herum angeordnet sind. Die zumindest zwei Bereiche des zumindest einen Abdeckelements sind vorzugsweise über einen Winkelbereich insbesondere von mehr als 180 °, bevorzugt von mehr als 270 ° und besonders bevorzugt von zumindest nahezu 360 ° angeordnet. Die zumindest zwei Bereiche des zumindest einen Abdeckelements decken zusammengenommen vorzugsweise einen Winkelbereich insbesondere von zumindest 90 °, vorzugsweise von zumindest 120 °und besonders bevorzugt von zumindest 180 ° um das zumindest eine Sicherungselement herum ab.

Zudem wird vorgeschlagen, dass das zumindest eine Abdeckelement zumindest teilweise fest mit der zumindest einen Saug- und/oder Spüleinheit verbunden ist. Dadurch kann eine bevorzugt zuverlässige Ausgestaltung der Befestigungseinheit erreicht werden. Das zumindest eine Abdeckelement ist vorzugsweise zumindest teilweise stoffschlüssig mit der zumindest einen Saug- und/oder Spüleinheit verbunden. Das zumindest eine Abdeckelement ist besonders bevorzugt zumindest teilweise mit der zumindest einen Saug- und/oder Spüleinheit verschweißt, insbesondere durch Laserschweißen. Es ist jedoch auch denkbar, dass das zumindest eine Abdeckelement und die zumindest eine Saug- und/oder Spüleinheit zumindest teilweise auf eine andere, einem Fachmann als sinnvoll erscheinende Weise, beispielsweise durch einen Kraftschluss oder durch eine Löt- oder Klebeverbindung, verbunden sind. Unter "fest verbunden" soll in diesem Zusammenhang insbesondere verstanden werden, dass das zumindest eine Abdeckelement und zumindest ein Teil der zumindest einen Saug- und/oder Spüleinheit verliersicher und, insbesondere unter Vermeidung einer Beschädigung und/oder Zerstörung zumindest eines der verbundenen Bauteile, untrennbar gekoppelt sind.

Ferner wird vorgeschlagen, dass das zumindest eine Abdeckelement zumindest teilweise aus Metall gebildet ist. Dadurch kann eine vorteilhaft robuste und zudem kostengünstige Ausgestaltung des zumindest einen Abdeckelements erreicht werden. Es ist auch denkbar, dass das zumindest eine Abdeckelement, alternativ oder zusätzlich, zumindest teilweise aus einem anderen, einem Fachmann als sinnvoll erscheinenden Material, wie beispielsweise aus Kunststoff, gebildet ist.

Des Weiteren wird vorgeschlagen, dass das zumindest eine Abdeckelement zumindest teilweise topfförmig ausgebildet ist. Dadurch kann eine konstruktiv einfache und vorteilhaft kompakte Ausgestaltung des zumindest einen Abdeckelements erreicht werden. Unter "topfförmig" soll in diesem Zusammenhang insbesondere verstanden werden, dass das zumindest eine Abdeckelement zumindest einen Bereich, der eine zumindest nahezu eben ausgebildete Haupterstreckungsfläche aufweist, und zumindest einen Bereich, der eine zumindest teilweise gekrümmt ausgebildete Haupterstreckungsfläche aufweist, umfasst, wobei Flächennormalen der Haupterstreckungsflächen der zumindest zwei Bereiche senkrecht zueinander verlaufen.

Dadurch, dass das zumindest eine Abdeckelement zumindest eine Bedienausnehmung zur Bedienung des zumindest einen Sicherungselements aufweist, kann eine vorteilhaft bedienerfreundliche und bevorzugt komfortable Bedienung bzw. Betätigung des zumindest einen Sicherungselements erreicht werden. Ferner kann eine bevorzugt leichte Ausgestaltung des zumindest einen Abdeckelements erreicht werden. Die zumindest eine Bedienausnehmung wird zumindest teilweise von dem zumindest einen Abdeckelement begrenzt. Unter einer "Bedienausnehmung" soll insbesondere ein, insbesondere materialfreier, Bereich des zumindest einen Abdeckelements verstanden werden, der dazu vorgesehen ist, eine Kontaktierung des zumindest einen Sicherungselements durch den Bediener des Endoskopiechirurgieinstruments zu einer Bedienung bzw. Betätigung des zumindest einen Sicherungselements zu ermöglichen. Die zumindest eine Bedienausnehmung weist vorzugsweise Abmessungen auf, die eine Bedienung bzw. Betätigung des zumindest einen Sicherungselements mit einem Finger ermöglichen.

Ferner wird vorgeschlagen, dass das zumindest eine Sicherungselement in einem montierten Zustand der zumindest einen Befestigungseinheit zumindest teilweise zu einer werkzeuglosen Sicherung und/oder Entsicherung des zumindest einen Endoskopieschafts mit der zumindest einen Saug- und/oder Spüleinheit vorgesehen ist. Dadurch kann eine vorteilhaft einfache und bevorzugt bedienerfreundliche Bedienung bzw. Betätigung des zumindest einen Sicherungselements erreicht werden.

Des Weiteren wird vorgeschlagen, dass das Endoskopiechirurgieinstrument zumindest eine Arretierungseinheit umfasst, die zu einer drehfesten Arretierung zumindest eines Teils der Saug- und/oder Spüleinheit relativ zu dem zumindest einen Endoskopieschaft vorgesehen ist. Dadurch kann ein vorteilhaft hoher Bedienkomfort des Endoskopiechirurgieinstruments, insbesondere während eines chirurgischen Eingriffs, erreicht werden. Unter einer "drehfesten Arretierung" soll in diesem Zusammenhang insbesondere eine Kopplung der zumindest einen Saug- und/oder Spüleinheit mit dem zumindest einen Endoskopieschaft unter Vermeidung einer Bewegung entlang zumindest einer Richtung, insbesondere einer Rotationsbewegung um eine parallel zur Längsrichtung des zumindest einen Endoskopieschafts verlaufende Achse, verstanden werden.

Das erfindungsgemäße Endoskopiechirurgieinstrument soll hierbei nicht auf die oben beschriebene Anwendung und Ausführungsform beschränkt sein. Insbesondere kann das erfindungsgemäße Endoskopiechirurgieinstrument zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein Endoskopiechirurgieinstrument in einem montierten Zustand in einer perspektivischen Seitenansicht,
- Fig. 2: das Endoskopiechirurgieinstrument in einem demontierten Zustand in einer Explosionsdarstellung,

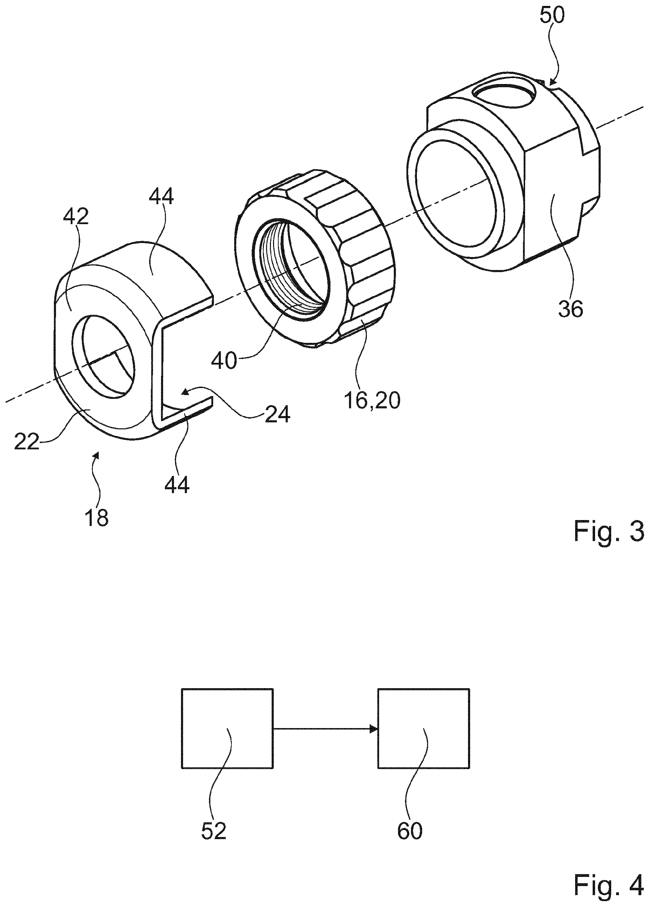

- Fig. 3: einen Teil einer Saug- und/oder Spüleinheit des Endoskopiechirurgieinstruments mit einer Befestigungseinheit in einer Explosionsdarstellung und
- Fig. 4: ein Ablaufdiagramm eines Verfahrens zur Montage des Endoskopiechirurgieinstruments.

### Beschreibung des Ausführungsbeispiels

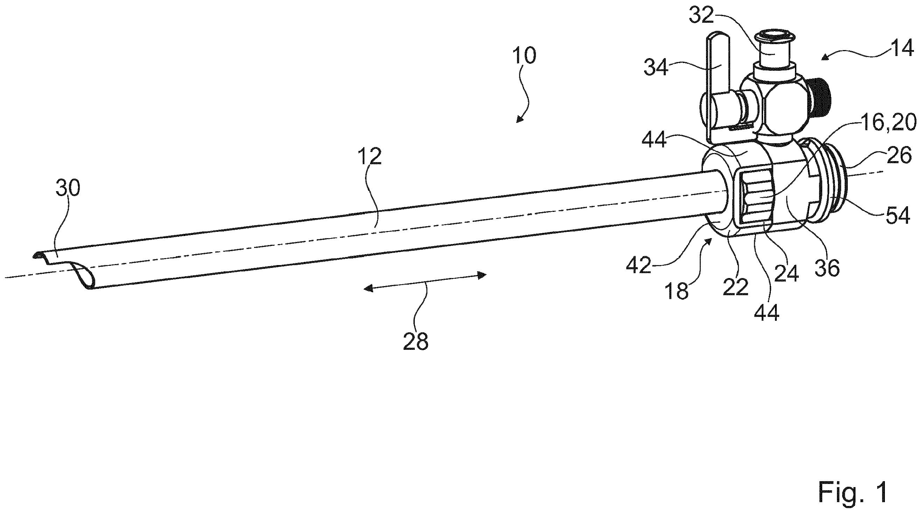

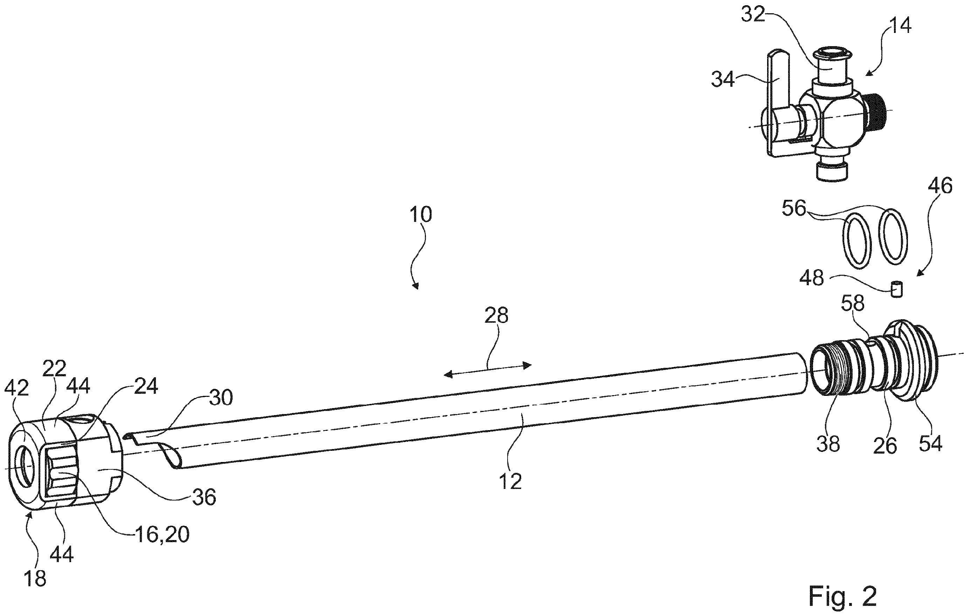

Die Figuren 1 und 2 zeigen ein Endoskopiechirurgieinstrument 10 mit zumindest einer Saug- und/oder Spüleinheit 14 und mit zumindest einem Endoskopieschaft 12. Das Endoskopiechirurgieinstrument 10 umfasst eine Saug- und/oder Spüleinheit 14 und einen Endoskopieschaft 12. Der Endoskopieschaft 12 ist aus Metall gebildet. Der Endoskopieschaft 12 ist rohrförmig ausgebildet. Der Endoskopieschaft 12 weist einen Außendurchmesser von 8 mm auf. Der Endoskopieschaft 12 weist einen Innendurchmesser von 7,2 mm auf. Der Endoskopieschaft 12 weist eine Länge von 150 mm auf. Der Endoskopieschaft 12 ist als starrer Endoskopieschaft ausgebildet. Der Endoskopieschaft 12 weist eine schnabelförmige Spitze 30 auf. Es sind jedoch auch andere, einem Fachmann als sinnvoll erscheinende Ausgestaltungen und/oder Abmessungen des Endoskopieschafts 12 denkbar. Das Endoskopiechirurgieinstrument 10 weist zumindest ein Kopplungselement 26 auf, das mit dem Endoskopieschaft 12 verbunden ist und das zu einer Kopplung des Endoskopieschafts 12 mit der Saug- und/oder Spüleinheit 14 vorgesehen ist. Das Endoskopiechirurgieinstrument 10 weist genau ein Kopplungselement 26 auf. Es ist jedoch auch denkbar, dass das Endoskopiechirurgieinstrument 10 mehr als ein Kopplungselement 26 aufweist. Das Kopplungselement 26 ist, in einer Längsrichtung 28 des Endoskopieschafts 12 betrachtet, an einem der schnabelförmigen Spitze 30 abgewandten Ende des Endoskopieschafts 12 angeordnet. Das Kopplungselement 26 ist fest mit dem Endoskopieschaft 12 verbunden. Das Kopplungselement 26 ist stoffschlüssig mit dem Endoskopieschaft 12 verbunden. Das Kopplungselement 26 ist durch eine Schweißverbindung mit dem Endoskopieschaft 12 verbunden. Das Kopplungselement 26 ist durch eine Laserschweißverbindung mit dem Endoskopieschaft 12 verbunden.

Die Saug- und/oder Spüleinheit 14 umfasst zumindest ein Anschlusselement 32 zu einem Anschluss eines Schlauchs. Die Saug- und/oder Spüleinheit 14 weist genau ein Anschlusselement 32 auf, das zum Anschluss eines Schlauchs vorgesehen ist. Es ist jedoch auch denkbar, dass die Saug- und/oder Spüleinheit 14 mehr als ein Anschlusselement 32, insbesondere zwei Anschlusselemente 32, aufweist. Die Saug- und/oder Spüleinheit 14 weist zudem zumindest ein Einstellelement 34 auf, das zu einer Einstellung einer Durchflussmenge durch die Saug- und/oder Spüleinheit 14 durch einen Bediener des Endoskopiechirurgieinstruments 10 vorgesehen ist. Das Einstellelement 34 ist als mechanisches Einstellelement ausgebildet. Das Einstellelement 34 ist von einem drehbar gelagerten Einstellhebel gebildet. Die Saug- und/oder Spüleinheit 14 weist zumindest ein Aufnahmeelement 36 auf, das zu einer Koppelung mit dem Endoskopieschaft 12 vorgesehen ist. Die Saug- und/oder Spüleinheit 14 weist genau ein Aufnahmeelement 36 auf. Es ist jedoch auch denkbar, dass die Saug- und/oder Spüleinheit 14 mehr als ein Aufnahmeelement 36 aufweist. Das Aufnahmeelement 36 der Saug- und/oder Spüleinheit 14 ist korrespondierend zu dem mit dem Endoskopieschaft 12 verbundenen Kopplungselement 26 ausgebildet. Das Aufnahmeelement 36 ist als Luer-Aufnahme ausgebildet.

Das Aufnahmeelement 36, das Einstellelement 34 und das Anschlusselement 32 der Saug- und/oder Spüleinheit 14 sind aus Metall gebildet. Das Aufnahmeelement 36, das Einstellelement 34 und das Anschlusselement 32 der Saug- und/oder Spüleinheit 14 sind aus Edelstahl gebildet. Das Aufnahmeelement 36, das Einstellelement 34 und das Anschlusselement 32 der Saug- und/oder Spüleinheit 14 bilden eine Montageeinheit. Das Aufnahmeelement 36, das Einstellelement 34 und das Anschlusselement 32 der Saug- und/oder Spüleinheit 14 sind fest verbunden. Das Aufnahmeelement 36 und das Anschlusselement 32 der Saug- und/oder Spüleinheit 14 sind stoffschlüssig verbunden. Es ist aber auch denkbar, dass das Aufnahmeelement 36 und das Anschlusselement 32 der Saug- und/oder Spüleinheit 14 zumindest teilweise form- und/oder kraftschlüssig verbunden sind. Das Aufnahmeelement 36 und das Anschlusselement 32 der Saug- und/oder Spüleinheit 14 sind verschweißt. Das Aufnahmeelement 36 und das Anschlusselement 32 der Saug- und/oder Spüleinheit 14 sind über eine Laserschweißverbindung verbunden. Es ist jedoch auch denkbar, dass das Aufnahmeelement 36 und das Anschlusselement 32 der Saug- und/oder Spüleinheit 14 auf eine andere, einem Fachmann als sinnvoll erscheinende Weise, wie beispielsweise über eine Klebeverbindung oder eine Pressverbindung, verbunden sind. In einem montierten Zustand umgreift das Aufnahmeelement 36 das Kopplungselement 26, das mit dem Endoskopieschaft 12 verbunden ist, zumindest teilweise. In einem montierten Zustand umgreift das Aufnahmeelement 36 das Kopplungselement 26 in einer senkrecht zur Längsrichtung 28 des Endoskopieschafts 12 angeordneten Ebene vollständig.

Das Endoskopiechirurgieinstrument 10 weist zudem zumindest ein Sicherungselement 16 auf, das dazu vorgesehen ist, den zumindest einen Endoskopieschaft 12 an der zumindest einen Saug- und/oder Spüleinheit 14 in einem gekoppelten Zustand zu sichern. Das Endoskopiechirurgieinstrument 10 weist genau ein Sicherungselement 16 zu einer Sicherung des Endoskopieschafts 12 relativ zur Saug- und/oder Spüleinheit 14 auf. Es ist aber auch denkbar, dass das Endoskopiechirurgieinstrument 10 mehr als ein Sicherungselement 16 umfasst. Das Sicherungselement 16 ist als Sicherungsmutter 20 ausgebildet. Das Sicherungselement 16 ist zu einer Sicherung der Saug- und/oder Spüleinheit 14 relativ zu dem Endoskopieschaft 12 in Längsrichtung 28 des Endoskopieschafts 12 vorgesehen. Das Sicherungselement 16 ist aus Metall gebildet. Das Sicherungselement 16 ist werkzeuglos betätigbar ausgebildet. Zur Sicherung der Saug- und/oder Spüleinheit 14 relativ zu dem Endoskopieschaft 12 wird das Sicherungselement 16 auf ein Gewinde 38 aufgeschraubt. Das Kopplungselement 26, das mit dem Endoskopieschaft 12 verbunden ist, weist das Gewinde 38 auf. Das Gewinde 38 ist als Außengewinde ausgebildet. Das Sicherungselement 16 weist ein Innengewinde 40 auf.

Das Endoskopiechirurgieinstrument 10 weist zudem zumindest eine Befestigungseinheit 18 auf, die zu einer verliersicheren Befestigung des Sicherungselements 16 an der Saug- und/oder Spüleinheit 14 vorgesehen ist. Das Endoskopiechirurgieinstrument 10 weist genau eine Befestigungseinheit 18 auf. Die Befestigungseinheit 18 umfasst zumindest ein Abdeckelement 22, das das Sicherungselement 16 in einem montierten Zustand zumindest teilweise umschließt. Die Befestigungseinheit 18 umfasst genau ein Abdeckelement 22. Es ist jedoch auch denkbar, dass die Befestigungseinheit 18 mehr als ein Abdeckelement 22 umfasst. Das Abdeckelement 22 umschließt das Sicherungselement 16 in zumindest einer Ebene teilweise. Das Abdeckelement 22 umschließt das Sicherungselement 16 in zumindest einer Ebene, die senkrecht zu einer Drehachse des Sicherungselements 16 angeordnet ist, teilweise. Die Drehachse des Sicherungselements 16 verläuft in einem montierten Zustand des Endoskopiechirurgieinstruments 10 parallel zur Längsrichtung 28 des Endoskopieschafts 12. Das Abdeckelement 22 ist zumindest teilweise aus Metall gebildet. Das Abdeckelement 22 ist vollständig aus Metall gebildet. Es ist jedoch auch denkbar, dass das Abdeckelement 22 aus einem anderen, einem Fachmann als sinnvoll erscheinenden Material, wie beispielsweise aus Kunststoff, gebildet ist. Das Abdeckelement 22 ist zumindest teilweise fest mit der Saug- und/oder Spüleinheit 14 verbunden. Das Abdeckelement 22 ist fest mit der Saug- und/oder Spüleinheit 14 verbunden. Das Abdeckelement 22 ist stoffschlüssig mit der Saug- und/oder Spüleinheit 14 verbunden. Das Abdeckelement 22 ist mit der Saug- und/oder Spüleinheit 14 verschweißt. Das Abdeckelement 22 ist durch Laserschweißen mit der Saug- und/oder Spüleinheit 14 verbunden. Das Abdeckelement 22 ist mit dem Aufnahmeelement 36 der Saug- und/oder Spüleinheit 14 verbunden.

Das Abdeckelement 22 ist zumindest teilweise topfförmig ausgebildet. Das Abdeckelement 22 weist zumindest eine Bedienausnehmung 24 zur Bedienung des Sicherungselements 16 auf. Das Abdeckelement 22 weist zwei Bedienausnehmungen 24 auf. Es jedoch auch eine abweichende Anzahl von Bedienausnehmungen 24 denkbar. Das Abdeckelement 22 weist einen ersten Bereich 42 und zwei weitere Bereiche 44 auf. Der erste Bereich 42 weist eine kreisringförmige Kontur auf. Der erste Bereich 42 ist plattenförmig ausgebildet. Die zwei weiteren Bereiche 44 des Abdeckelements 22 sind an den ersten Bereich 42 anschließend angeordnet. Die zwei weiteren Bereiche 44 des Abdeckelements 22 sind an den ersten Bereich 42 direkt anschließend angeordnet. Der erste Bereich 42 und die zwei weiteren Bereiche 44 des Abdeckelements 22 sind einstückig ausgebildet. Die zwei weiteren Bereiche 44 des Abdeckelements 22 sind identisch ausgebildet. Die weiteren Bereiche 44 sind jeweils plattenförmig und gewölbt ausgebildet. Die weiteren Bereiche 44 weisen jeweils eine Längsrichtung auf, die parallel zu einer Flächennormalen des ersten Bereichs 42 des Abdeckelements 22 verläuft. In einem montierten Zustand des Endoskopiechirurgieinstruments 10 verläuft die Flächennormale einer Haupterstreckungsfläche des ersten Bereichs 42 des Abdeckelements 22 parallel zur Längsrichtung 28 des Endoskopieschafts 12. Die weiteren Bereiche 44 sind jeweils um eine Achse gewölbt ausgebildet, die parallel zur Längsrichtung der weiteren Bereiche 44 bzw. zur Flächennormalen der Haupterstreckungsfläche des ersten Bereichs 42 verläuft. Die weiteren Bereiche 44 sind auf einer Kreislinie angeordnet. Die weiteren Bereiche 44 sind auf einer Umfangskreislinie des ersten Bereichs 42 angeordnet. Die weiteren Bereiche 44 sind, entlang eines Durchmessers des ersten Bereichs 42 betrachtet, gegenüberliegend angeordnet. Die weiteren Bereiche 44 sind in einer Umfangsrichtung des Abdeckelements 22 beabstandet angeordnet. Die weiteren Bereiche 44 begrenzen die Bedienausnehmungen 24 in Umfangsrichtung. In einem montierten Zustand der Befestigungseinheit 18 ist das Sicherungselement 16 zwischen den weiteren Bereichen 44 des Abdeckelements 22 angeordnet. Das Abdeckelement 22 ist jeweils an einem dem ersten Bereich 42 abgewandten Ende der weiteren Bereiche 44 mit der Saug- und/oder Spüleinheit 14 verbunden.

Das Abdeckelement 22 weist einen größten Abstand zwischen den weiteren Bereichen 44 von 16,3 mm auf. Das Abdeckelement 22 weist eine Höhe, senkrecht zu dem Abstand zwischen den weiteren Bereichen 44 betrachtet, von 8,3 mm auf. Das Abdeckelement 22 weist eine kleinste Breite, senkrecht zur Höhe des Abdeckelements 22 betrachtet, von 13 mm auf. Der erste Bereich 42 des Abdeckelements 22 weist eine Stärke von 0,4 mm auf. Die weiteren Bereiche 44 des Abdeckelements 22 weisen jeweils eine Höhe, parallel zur Flächennormalen der Haupterstreckungsfläche des ersten Bereichs 42 betrachtet, von 6,9 mm auf. Eine kreisförmige Ausnehmung in dem ersten Bereich 42 des Abdeckelements 22 weist einen Durchmesser von 8,2 mm auf. Das Abdeckelement 22 weist, parallel zur Haupterstreckungsfläche des ersten Bereichs 42 betrachtet, eine größte Erstreckung von 17,5 mm auf. Es sind jedoch auch andere, einem Fachmann als sinnvoll erscheinende Abmessungen denkbar.

In einem montierten Zustand der Befestigungseinheit 18 ist das Sicherungselement 16 relativ zu dem Abdeckelement 22 der Befestigungseinheit 18 und relativ zu dem Aufnahmeelement 36 der Saug- und/oder Spüleinheit 14 verliersicher gelagert. In einem montierten Zustand der Befestigungseinheit 18 ist das Sicherungselement 16 relativ zu dem Abdeckelement 22 der Befestigungseinheit 18 und relativ zu dem Aufnahmeelement 36 der Saug- und/oder Spüleinheit 14 bewegbar gelagert. In einem montierten Zustand der Befestigungseinheit 18 ist das Sicherungselement 16 relativ zu dem Abdeckelement 22 der Befestigungseinheit 18 und relativ zu dem Aufnahmeelement 36 der Saug- und/oder Spüleinheit 14 drehbar gelagert. Die Befestigungseinheit 18, das Sicherungselement 16 und die Saug- und/oder Spüleinheit 14 bilden eine Montageeinheit. Das Sicherungselement 16 ist in einem montierten Zustand der Befestigungseinheit 18 zumindest teilweise zu einer werkzeuglosen Sicherung und/oder Entsicherung des Endoskopieschafts 12 mit der Saug- und/oder Spüleinheit 14 vorgesehen. Das Sicherungselement 16 ist in einem montierten Zustand der Befestigungseinheit 18 zu einer werkzeuglosen Sicherung und/oder Entsicherung vorgesehen.

Das Endoskopiechirurgieinstrument 10 umfasst zudem zumindest eine Arretierungseinheit 46, die zu einer drehfesten Arretierung der Saug- und/oder Spüleinheit 14 relativ zu dem Endoskopieschaft 12 vorgesehen ist. Das Endoskopiechirurgieinstrument 10 umfasst genau eine Arretierungseinheit 46. Es ist jedoch auch denkbar, dass das Endoskopiechirurgieinstrument 10 mehr als eine Arretierungseinheit 46 aufweist. Die Arretierungseinheit 46 ist zu einer drehfesten Arretierung des Aufnahmeelements 36 der Saug- und/oder Spüleinheit 14 relativ zu dem Kopplungselement 26, das fest mit dem Endoskopieschaft 12 verbunden ist, vorgesehen. Die Arretierungseinheit 46 weist zumindest ein Arretierungselement 48 auf. Die Arretierungseinheit 46 weist genau ein Arretierungselement 48 auf. Es ist jedoch auch denkbar, dass die Arretierungseinheit 46 mehr als ein Arretierungselement 48 aufweist. Das Arretierungselement 48 ist zylinderförmig ausgebildet. Das Arretierungselement 48 ist stiftförmig ausgebildet. Das Arretierungselement 48 weist einen Durchmesser von 2 mm auf. Das Arretierungselement 48 weist eine Länge von 2,7 mm auf. Das Arretierungselement 48 ist fest mit dem Kopplungselement 26, das mit dem Endoskopieschaft 12 gekoppelt ist, verbunden. Das Arretierungselement 48 ist stoffschlüssig mit dem Kopplungselement 26 verbunden. Das Arretierungselement 48 ist mit dem Kopplungselement 26 verschweißt. Eine Rotationssymmetrieachse des Arretierungselements 48 ist in einem montierten Zustand senkrecht zur Längsrichtung 28 des Endoskopieschafts 12 angeordnet. In das Aufnahmeelement 36 der Saug- und/oder Spüleinheit 14 ist zumindest eine korrespondierend zu dem Arretierungselement 48 ausgebildete Arretierungsausnehmung 50 eingebracht. In das Aufnahmeelement 36 der Saug- und/oder Spüleinheit 14 sind zwei, in Umfangsrichtung versetzt angeordnete Arretierungsausnehmungen 50 eingebracht. Es ist jedoch auch eine andere, einem Fachmann als sinnvoll erscheinende Anzahl von Arretierungsausnehmungen 50 denkbar. Die zwei Arretierungsausnehmungen 50 sind, in Umfangsrichtung des Aufnahmeelements 36 betrachtet, um 90 ° versetzt angeordnet.

Es ist alternativ auch denkbar, dass das Endoskopiechirurgieinstrument 10 keine Arretierungseinheit 46 aufweist und die Saug- und/oder Spüleinheit 14 in einem montierten Zustand relativ zu dem Endoskopieschaft 12 drehbar gelagert ist.

Ein Verfahren zu einer Montage des Endoskopiechirurgieinstruments 10 umfasst zumindest einen Verfahrensschritt 52, in dem die Saug- und/oder Spüleinheit 14 relativ zu dem Endoskopieschaft 12 positioniert wird. In dem Verfahrensschritt 52 wird das Aufnahmeelement 36 der Saug- und/oder Spüleinheit 14 in Längsrichtung 28, von der Spitze 30 des Endoskopieschafts 12 zu dem Kopplungselement 26 hin, auf den Endoskopieschaft 12 aufgeschoben. Das Aufnahmeelement 36 der Saug- und/oder Spüleinheit 14 umgreift dabei den Endoskopieschaft 12. Die Saug- und/oder Spüleinheit 14 wird in Längsrichtung 28 entlang des Endoskopieschafts 12 verschoben, bis das Aufnahmeelement 36 der Saug- und/oder Spüleinheit 14 an einen Anschlag 54 des Kopplungselements 26, der auf einer dem Endoskopieschaft 12 abgewandten Ende des Kopplungselements 26 angeordnet ist, anstößt. Der Anschlag 54 ist einstückig mit dem Kopplungselement 26 ausgebildet. Der Anschlag 54 begrenzt eine Bewegung der Saug- und/oder Spüleinheit 14 in eine Richtung parallel zur Längsrichtung 28 des Endoskopieschafts 12, von der Spitze 30 des Endoskopieschafts 12 zum Kopplungselement 26 hin betrachtet.

Mit dem Kopplungselement 26 ist in einem montierten Zustand ferner zumindest ein Dichtungselement 56 gekoppelt, das in einem montierten Zustand der Saug- und/oder Spüleinheit 14 mit dem Aufnahmeelement 36 der Saug- und/oder Spüleinheit 14 abdichtet. Mit dem Kopplungselement 26 sind zwei Dichtungselemente 56 gekoppelt. Es ist jedoch auch denkbar, dass eine andere Anzahl von Dichtungselementen 56 vorgesehen ist. Die Dichtungselemente 56 sind jeweils von einem Dichtungsring gebildet. Die Dichtungselemente 56 sind jeweils von einem O-Ring gebildet. In das Kopplungselement 26 ist zumindest eine Durchflussöffnung 58 eingebracht. In das Kopplungselement 26 ist genau eine Durchflussöffnung 58 eingebracht. Es ist jedoch auch denkbar, dass in das Kopplungselement 26 mehr als eine Durchflussöffnung 58 eingebracht ist. Die Durchflussöffnung 58 erstreckt sich durch eine gesamte Materialstärke des Kopplungselements 26 und ist dazu vorgesehen, in einem Betriebszustand des Endoskopiechirurgieinstrument 10 einen Flüssigkeitstransport aus dem Endoskopieschaft 12 zu dem Anschlusselement 32 der Saug- und/oder Spüleinheit 14 und/oder von dem Anschlusselement 32 der Saug- und/oder Spüleinheit 14 in den Endoskopieschaft 12 zu ermöglichen. Die Durchflussöffnung 58 ist, in Längsrichtung 28 des Endoskopieschafts 12 betrachtet, zwischen den zwei Dichtungselementen 56 angeordnet.

Das Verfahren umfasst zumindest einen weiteren Verfahrensschritt 60, in dem die Saug- und/oder Spüleinheit 14 um eine Längsachse des Endoskopieschafts 12, die parallel zur Längsrichtung 28 verläuft, vom Bediener des Endoskopiechirurgieinstruments 10 in eine gewünschte Position gedreht und in dieser Position mittels des Sicherungselements 16 gesichert wird. Dazu dreht der Bediener die Saug- und/oder Spüleinheit 14, bis eine der Arretierungsausnehmungen 50 im Aufnahmeelement 36 in Deckung mit dem Arretierungselement 48 kommt. Anschließend wird das Sicherungselement 16 auf das Gewinde 38 des Kopplungselements 26 aufgeschraubt. Dadurch wird die Saug- und/oder Spüleinheit 14 in eine Richtung parallel zur Längsrichtung 28 des Endoskopieschafts 12, von dem Kopplungselement 26 zur Spitze 30 des Endoskopieschafts 12 hin betrachtet, und zudem durch das Ineinandergreifen der Arretierungsausnehmung 50 und des Arretierungselements 48 in Umfangsrichtung gesichert. Zu einem Wechseln einer Positionierung der Saug- und/oder Spüleinheit 14 relativ zu dem Endoskopieschaft 12 in Umfangsrichtung wird das Sicherungselement 16 wieder gelöst, die Saug- und/oder Spüleinheit 14 relativ zu dem Endoskopieschaft 12 in Umfangsrichtung gedreht, bis die andere der Arretierungsausnehmungen 50 in Deckung mit dem Arretierungselement 48 kommt, und anschließend das Sicherungselement 16 erneut auf das Gewinde 38 des Kopplungselements 26 aufgeschraubt.

## Patentansprüche

1. Endoskopiechirurgieinstrument mit zumindest einem Endoskopieschaft (12), mit zumindest einer Saug- und/oder Spüleinheit (14), mit zumindest einem Sicherungselement (16), das dazu vorgesehen ist, den zumindest einen Endoskopieschaft (12) an der zumindest einen Saug- und/oder Spüleinheit (14) in einem gekoppelten Zustand zu sichern, und mit zumindest einer Befestigungseinheit (18), die zu einer verliersicheren Befestigung des zumindest einen Sicherungselements (16) an der zumindest einen Saug- und/oder Spüleinheit (14) vorgesehen ist,
**dadurch gekennzeichnet, dass**
die zumindest eine Befestigungseinheit (18) zumindest ein Abdeckelement (22) umfasst, das das zumindest eine Sicherungselement (16) in einem montierten Zustand zumindest teilweise umschließt, wobei das zumindest eine Abdeckelement (22) zumindest eine Bedienausnehmung (24) zur Bedienung des zumindest einen Sicherungselements (16) aufweist.

2. Endoskopiechirurgieinstrument nach Anspruch 1,
**dadurch gekennzeichnet, dass**
das zumindest eine Sicherungselement (16) als Sicherungsmutter (20) ausgebildet ist.

3. Endoskopiechirurgieinstrument nach Anspruch 1 oder 2,
**dadurch gekennzeichnet, dass**
das zumindest eine Abdeckelement (22) zumindest teilweise fest mit der zumindest einen Saug- und/oder Spüleinheit (14) verbunden ist.

4. Endoskopiechirurgieinstrument nach einem der vorhergehenden Ansprüche,
**dadurch gekennzeichnet, dass**
das zumindest eine Abdeckelement (22) zumindest teilweise aus Metall gebildet ist.

5. Endoskopiechirurgieinstrument nach einem der vorhergehenden Ansprüche,
**dadurch gekennzeichnet, dass**
das zumindest eine Abdeckelement (22) zumindest teilweise topfförmig ausgebildet ist.

6. Endoskopiechirurgieinstrument nach einem der vorhergehenden Ansprüche,
**dadurch gekennzeichnet, dass**
das zumindest eine Sicherungselement (16) in einem montierten Zustand der zumindest einen Befestigungseinheit (18) zumindest teilweise zu einer werkzeuglosen Sicherung und/oder Entsicherung des zumindest einen Endoskopieschafts (12) mit der zumindest einen Saug- und/oder Spüleinheit (14) vorgesehen ist.

7. Endoskopiechirurgieinstrument nach einem der vorhergehenden Ansprüche, **gekennzeichnet durch**
zumindest eine Arretierungseinheit (46), die zu einer drehfesten Arretierung zumindest eines Teils der Saug- und/oder Spüleinheit (14) vorgesehen ist.

## Claims

1. Endoscopic surgical instrument with at least one endoscopy shaft (12), with at least one suction and/or rinse unit (14), with at least one securing element (16) that is configured, in a coupled state, to secure the at least one endoscopy shaft (12) on the at least one suction and/or rinse unit (14), and with at least one fixation unit (18) that is configured for a captive fixation of the at least one securing element (16) on the at least one suction and/or rinse unit (14),
**characterised in that** the at least one fixation unit (18) comprises at least one cover element (22) which, in an assembled state, encompasses the at least one securing element (16) at least partly, wherein the at least one cover element (22) comprises at least one operational recess (24) for an operation of the at least one securing element (16).

2. Endoscopic surgical instrument according to claim 1, **characterised in that** the at least one securing element (16) is embodied as a securing nut (20).

3. Endoscopic surgical instrument according to claim 1 or 2, **characterised in that** the at least one cover element (22) is fixedly connected with the at least one suction and/or rinse unit (14) at least partly.

4. Endoscopic surgical instrument according to one of the preceding claims, **characterised in that** the at least one cover element (22) is embodied at least partly of metal.

5. Endoscopic surgical instrument according to one of the preceding claims, **characterised in that** the at least one cover element (22) is embodied to be at least partly pot-shaped.

6. Endoscopic surgical instrument according to one of the preceding claims, **characterised in that** the at least one securing element (16) is configured, in an assembled state of the at least one fixation unit (18), at least partly for a tool-free securing and/or release of the at least one endoscopy shaft (12) with the at least one suction and/or rinse unit (14).

7. Endoscopic surgical instrument according to one of the preceding claims, **characterised by** at least one locking unit (46), which is configured for a rotationally fixed locking of at least a portion of the suction and/or rinse unit (14).

## Revendications

1. Instrument de chirurgie endoscopique avec au moins un manche endoscopique (12), avec au moins une unité de succion et/ou rinçage (14), avec au moins un élément de sécurisation (16) prévu à sécuriser l'au moins un manche endoscopique (12) sur l'au moins une unité de succion et/ou rinçage (14) en état raccordé, et avec au moins une unité de fixation (18) prévue fixer imperdablement l'au moins un élément de sécurisation (16) sur l'au moins une unité de succion et/ou rinçage (14),
**caractérisé en ce que** l'au moins une unité de fixation (18) comporte au moins un élément de couverture (22) entourant, en état monté, l'au moins un élément de sécurisation (16) au moins partiellement,
l'au moins un élément de couverture (22) comportant au moins une échancrure à maniement (24) pour un maniement de l'au moins un élément de sécurisation (16).

2. Instrument de chirurgie endoscopique selon la revendication 1,
**caractérisé en ce que** l'au moins un élément de sécurisation (16) est réalisé comme écrou de sécurisation.

3. Instrument de chirurgie endoscopique selon l'une quelconque des revendications 1 ou 2,
**caractérisé en ce que** l'au moins un élément de couverture (22) est au moins partiellement raccordé fixement avec l'au moins une unité de succion et/ou rinçage (14).

4. Instrument de chirurgie endoscopique selon l'une quelconque des revendications précédentes,
**caractérisé en ce que** l'au moins un élément de couverture (22) est réalisé au moins partiellement de métal.

5. Instrument de chirurgie endoscopique selon l'une quelconque des revendications précédentes,
**caractérisé en ce que** l'au moins un élément de couverture (22) est réalisé au moins partiellement en forme de casserole.

6. Instrument de chirurgie endoscopique selon l'une quelconque des revendications précédentes,
**caractérisé en ce que** l'au moins un élément de sécurisation (16) est prévu, en état monté de l'au moins une unité de fixation (18), au moins partiellement pour une sécurisation sans outil et/ou un déblocage sans outil de l'au moins un manche endoscopique (12) avec l'au moins une unité de succion et/ou rinçage (14).

7. Instrument de chirurgie endoscopique selon l'une quelconque des revendications précédentes,
**caractérisé par** au moins une unité d'arrêt (46) prévue pour un arrêt fixé en rotation au moins d'une partie de l'unité de succion et/ou rinçage (14).
